# EUROPEAN PATENT SPECIFICATION

(11) **EP 2 635 332 B1**
(45) Date of publication and mention of the grant of the patent: **13.08.2014**
(21) Application number: 11781480.6
(22) Date of filing: 31.10.2011
(51) Int. Cl.: A61M 5/28, A61M 5/32, A61M 5/24

(54) **MEDICATED MODULE WITH DEFORMABLE MEMBRANE**
EIN ARZNEISTOFF ENTHALTENDES MODUL MIT VERFORMBARER MEMBRAN
MODULE MÉDICAMENTEUX COMPORTANT UNE MEMBRANE DÉFORMABLE

(30) Priority: 03.11.2010 EP 10189777
(43) Date of publication of application: 11.09.2013
(73) Proprietor: Sanofi-Aventis Deutschland GmbH, 65929 Frankfurt am Main (DE)
(72) Inventor: DAVIES, James Alexander, Leamington Spa Warwickshire CV32 7XB (GB); SANDERS, David, Warwick CV34 5LJ (GB); MOORE, David, Elmesthorpe Leicester Leicestershire LE9 7SA (GB); WIMPENNY, Steven, Leamington Spa Warwickshire CV32 6ES (GB)
(74) Representative: Keil & Schaafhausen Patent- und Rechtsanwälte
(86) International application number: PCT/EP2011/069095
(87) International publication number: WO 2012/059450

(56) References cited:
- WO-A2-2008/063439
- US-A- 5 102 388
- US-B1- 6 562 002

## Description

### Field of the Present Patent Application

This present patent application relates to medical devices and methods of delivering at least two drug agents from separate reservoirs using devices having only a single dose setting mechanism and a single dispense interface. A single delivery procedure initiated by the user causes a non-user settable dose of a second drug agent and a variable set dose of a first drug agent to be delivered to the patient. The drug agents may be available in two or more reservoirs, containers or packages, each containing independent (single drug compound) or pre-mixed (co-formulated multiple drug compounds) drug agents. Specifically, this application concerns a method and system for dispensing a non-user settable dose of one medicament followed by a user-settable dose of a primary medicament. In one arrangement, the proposed system and method concerns sequential dosing of two medicaments.

### Background

Certain disease states require treatment using one or more different medicaments. Some drug compounds need to be delivered in a specific relationship with each other in order to deliver the optimum therapeutic dose. The presently proposed devices and methods are of particular benefit where combination therapy is desirable, but not possible in a single formulation for reasons such as, but not limited to, stability, compromised therapeutic performance and toxicology.

For example, in some cases it might be beneficial to treat a diabetic with a long acting insulin and with a glucagon-like peptide-1 (GLP-1), which is derived from the transcription product of the proglucagon gene. GLP-1 is found in the body and is secreted by the intestinal L cell as a gut hormone. GLP-1 possesses several physiological properties that make it (and its analogs) a subject of intensive investigation as a potential treatment of diabetes mellitus.

There are a number of potential problems when delivering two active medicaments or "agents" simultaneously. The two active agents may interact with each other during the long-term, shelf life storage of the formulation. Therefore, it is advantageous to store the active components separately and combine them at the point of delivery, e.g. injection, needle-less injection, pumps, or inhalation. However, the process for combining the two agents needs to be simple and convenient for the user to perform reliably, repeatedly and safely.

A further problem is that the quantities and/or proportions of each active agent making up the combination therapy may need to be varied for each user or at different stages of their therapy. For example one or more active agents may require a titration period to gradually introduce a patient up to a "maintenance" dose. A further example would be if one active agent requires a non-adjustable fixed dose while the other is varied in response to a patient's symptoms or physical condition. This problem means that pre-mixed formulations of multiple active agents may not be suitable as these pre-mixed formulations would have a fixed ratio of the active components, which could not be varied by the healthcare professional or user.

Additional problems could arise where a multi-drug compound therapy is required, because many users cannot cope with having to use more that one drug delivery system or make the necessary accurate calculation of the required dose combination. This is especially true for users with dexterity or computational difficulties. In some circumstances it is also necessary to perform a priming procedure of the device and/or needle cannulae before dispensing the medicaments. Likewise, in some situations, it may be necessary to bypass one drug compound and to dispense only a single medicament from a separate reservoir. Further, for some drug combinations for which this delivery of two medicaments in a single injection step is desirable, it may be additionally desirable for the two medicaments to be delivered sequentially (i.e., one after the other, with minimal or no opportunity for mixing). Avoidance of mixing of the 2 drug formulations might have several advantages. For example, it is known that the pharmacokinetics of certain drugs is critically dependent on their concentration. By delivering 2 drugs sequentially with no mixing the optimal concentration of each drug for optimal pharmacokinetics can be maintained. In addition, certain drugs have to be formulated in particular solvent environments (e.g., a specific pH range) to remain in solution. By delivering 2 drugs sequentially with no mixing the optimal pH range and therefore solubility can be maintained during delivery.

Accordingly, there exists a need to provide devices and methods for the sequential delivery of two or more medicaments in a single injection or delivery step that is simple for the user to perform.

US 6,562,002 B1 discloses a single dose delivery device that combines a micro-injection device comprising a housing, which is shaped and sized to house a reagent bed and a porous compression component, with a diluent delivery device. During delivery, diluent flows from the diluent delivery device through a needle into a reagent chamber of the micro-injection device. Passage of the diluent through the reagent bed results in dissolution of the reagent. The reagent solution is provided by an injection needle.

The presently proposed devices and methods overcome the above-mentioned problems by providing separate storage containers for two or more active drug agents that are then delivered sequentially to the patient during a single delivery procedure. Beneficially, delivering the drugs sequentially may avoid or limit mixing of the drug agents until a point of delivery (i.e. in vivo). Setting a dose of one medicament automatically fixes or determines the dose of the second medicament (i.e., non-user settable). The proposed devices and methods also give the opportunity for varying the quantity of one or both medicaments. For example, one fluid quantity can be varied by changing the properties of the injection device (e.g., dialing a user variable dose or changing the device's "fixed" dose). The second fluid quantity can be changed by manufacturing a variety of secondary drug containing packages with each variant containing a different volume and/or concentration of the second active agent. The user or healthcare professional would then select the most appropriate secondary package or series or combination of series of different packages for a particular treatment regime. The proposed medicated module forms a self-contained reservoir in which a non-user-settable dose of a medicament may be stored.

These and other advantages will become evident from the following more detailed description of the invention.

### SUMMARY

The presently proposed devices and methods allow for complex combinations of multiple drug compounds within a single drug delivery system. Further, the presently proposed devices and methods allow the user to set and sequentially dispense at least two drug agents through one single dose setting mechanism and a single dispense interface. This single dose setter controls the mechanism of the device such that a predefined combination of the individual drug compounds is delivered when a single dose of one of the medicaments is set and dispensed through the single dispense interface.

By defining the therapeutic relationship between the individual drug compounds, the proposed delivery device and delivery methods help ensure that a patient/user receives the optimum therapeutic combination dose from a multi-drug compound device without the inherent risks associated with multiple inputs where the user has to calculate and set the correct dose combination every time they use the device. The medicaments can be fluids, defined herein as liquids or gases or powders that are capable of flowing and that change shape at a steady rate when acted upon by a force tending to change its shape.

Applicants' proposed concept is of particular benefit to users with dexterity or computational difficulties as the single input and associated predefined therapeutic profile removes the need for them to calculate their prescribed dose every time they use the device and the single input allows considerably easier setting and dispensing of the combined compounds.

In a preferred embodiment a master drug compound, such as insulin, contained within a multiple dose, user selectable drug delivery device could be used with a single use, user replaceable, medicated module that contains a single dose of a secondary medicament and the single dispense interface. When connected to the primary drug delivery device, the secondary compound is activated/delivered on dispense of the primary compound. Although the present application specifically mentions insulin, insulin analogs or insulin derivatives, and GLP-1 or GLP-1 analogs as two possible drug combinations, other drugs or drug combinations, such as an analgesics, hormones, beta agonists or corticosteroids, or a combination of any of the above-mentioned drugs could be used with our proposed method and system.

For the purposes of our proposed method and system the term "insulin" shall mean Insulin, insulin analogs, insulin derivatives or mixtures thereof, including human insulin or a human insulin analogs or derivatives. Examples of insulin analogs are, without limitation, Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin or Des(B30) human insulin. Examples of insulin derivatives are, without limitation, B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyhepta-decanoyl) human insulin.

As used herein the term "GLP-1" shall mean GLP-1, GLP-1 analogs, or mixtures thereof, including without limitation, exenatide (Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2), Exendin-3, Liraglutide, or AVE001 0 (H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Ser-Lys-Lys-Lys-Lys-Lys-Lys-NH2).

Examples of beta agonists are, without limitation, salbutamol, levosalbutamol, terbutaline, pirbuterol, procaterol, metaproterenol, fenoterol, bitolterol mesylate, salmeterol, formoterol, bambuterol, clenbuterol, indacaterol.

Hormones are for example hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

According to an embodiment, a medicated module is attachable to a drug delivery device, and the drug delivery device has a drug reservoir holding a first medicament.

The medicated module comprises a first needle, a second needle, a deformable membrane, and a second medicament. At least part of the deformable membrane is located between the first and second needle. Further, the second medicament is located on a distal side of the deformable membrane. After the medicated module is attached to the drug delivery device, (i) the first needle is in fluid communication with the drug reservoir and (ii) the deformable membrane prevents fluid communication between the first and second needle. During dosing, the first medicament flows through the first needle and deforms the deformable membrane, wherein deformation of the deformable membrane forces substantially all of the second medicament to flow through the second needle. After substantially all of the second medicament is forced to flow through the second needle, the second needle pierces the deformable membrane, thereby providing fluid communication between the first needle and the second needle. The first medicament may then be dispensed from the output needle.

According to an embodiment, the medicated module is attachable to a drug delivery device, wherein the drug delivery device has a drug reservoir holding a first medicament. The medicated module comprises a first needle, a second needle, a deformable membrane, and holds a second medicament. The second medicament may be located directly in a cavity formed between the deformable membrane and a lower or bottom surface of the module. Alternatively, the second medicament may be contained in a capsule, container, membrane, cusion, or the like. The capsule may be located in a cavity formed by a deformable membrane and a lower or bottom surface of the medicated module.

According to another embodiment, a drug delivery system to deliver a non-user settable dose of one medicament followed by a user-settable dose of a primary medicament through a single dose setter and a single dispense interface is provided. The drug delivery system includes a drug delivery device and a medicated module. The drug delivery device comprises (i) a housing including a single dose setter operably connected to a primary reservoir of medicament including the primary medicament and (ii) a dose button operably connected to the primary reservoir of medicament. The medicated module is attachable to the drug delivery device and includes a first needle, a second needle, a deformable membrane, and a second medicament. At least a portion of the deformable membrane is located between the first and second needle. Further, the second medicament is located on a distal side of the deformable membrane.

After the medicated module is attached to the drug delivery device, (i) the first needle is in fluid communication with the primary drug reservoir and (ii) the deformable membrane prevents fluid communication between the first and second needle. During a dose setting step, the first medicament flows through the first needle and deforms the deformable membrane, wherein deformation of the deformable membrane forces a substantial portion of the second medicament to flow through the second needle. After this portion of the second medicament is forced to flow through the second needle, the second needle pierces the deformable membrane, thereby opening up fluid communication between the first needle and the second needle.

According to yet another embodiment, a method of dispensing a non-user settable dose of one medicament followed by a user-settable dose of a primary medicament using a single dispense interface is provided. The method includes attaching a medicated module, such as the medicated modules discussed above, to a drug delivery device, such as the drug delivery device discussed above. The method further includes setting a dose of the primary medicament contained in the primary drug reservoir using a single dose setter of the drug delivery device. The method further includes activating a dose button on the drug delivery device to cause the set dose of the primary medicament from the primary drug reservoir to flow in the distal direction toward the deformable membrane. The primary medicament deforms the deformable membrane, and deformation of the membrane forces substantially all of the second medicament to flow through the second needle. After substantially all of the second medicament is forced to flow through the second needle, the second needle pierces the deformable membrane, thereby opening up fluid communication between the first needle and the second needle. The method then includes forcing the primary medicament to flow through the second needle.

A medicated module in accordance with Applicants' proposed concept can be designed for use with any drug delivery device with an appropriate compatible interface. However, it may be preferable to design the module in such a way as to limit its use to one exclusive primary drug delivery device (or family of devices) through employment of dedicated or coded features to prevent attachment of a non-appropriate medicated module to a non-matching device. In some situations it may be beneficial to ensure that the medicated module is exclusive to one drug delivery device while also permitting the attachment of a standard drug dispense interface to the device. This would allow the user to deliver a combined therapy when the module is attached, but would also allow delivery of the primary compound independently through a standard drug dispense interface in situations, such as, but not limited to, dose splitting or top-up of the primary compound.

A particular benefit of Applicants' method and system is that the method and system allow for sequential dosing of a first medicament and a second medicament through a single dispense interface. Thus, the method and system beneficially prevents or limits mixing of the first, primary medicament and the second medicament. This may be beneficial, for example, when mixing of medicaments prior to their delivery into an injection site negatively or detrimentally affects at least one of the medicaments.

In a preferred embodiment, the primary drug delivery device is used more than once and therefore is a multi-use device; however, the drug delivery device may also be a single use disposable device. Such a device may or may not have a replaceable reservoir of the primary drug compound, but the proposed concept is equally applicable to both scenarios. It is also possible to have a suite of different medicated modules for various conditions that could be prescribed as one-off extra medication to patients already using a standard drug delivery device. Should the patient attempt to reuse a previously used medicated module, features may be present that prevent reattachment to a primary drug delivery device or that prevent or discourage subsequent dosing through the needle via alternative means. For example, this module may include a locking needle guard that is activated after a user delivers a dose from the medicated module. Other means of alerting the user may include some (or all) of the following:

Physical prevention of medicated module re-attachment to the primary drug delivery device once the module has been used and removed.

Physical / hydraulic prevention of subsequent liquid flow through the drug dispense interface once it has been used.

Physical locking of the dose setter and/or dose button of the primary drug delivery device.

Visual warnings (e.g., change in color and/or warning text/indicia within an indication window on the module once insertion and/or fluid flow has occurred).

Tactile feedback (presence or absence of tactile features on the outer surface of the module hub following use).

A further proposed feature is that both medicaments are delivered via one injection needle and in one injection step. This offers a convenient benefit to the user in terms of reduced user steps compared to administering two separate injections. This convenience benefit may also result in improved compliance with the prescribed therapy, particularly for users who find injections unpleasant or who have computational or dexterity difficulties.

These as well as other advantages of various aspects of the present invention will become apparent to those of ordinary skill in the art by reading the following detailed description, with appropriate reference to the accompanying drawings.

### BRIEF DESCRIPTION OF THE DRAWINGS

Exemplary embodiments are described herein with reference to the drawings, in which:
Figure 1 illustrates a perspective view of one possible drug delivery device that can be used with Applicants' proposed medicated module;
Figure 2 illustrates a cross-sectional view of an exemplary medicated module attached to an exemplary drug delivery device;
Figure 3 illustrates a cross-sectional view of the exemplary medicated module and exemplary drug delivery device of Figure 2 after the deformable membrane is pierced;
Figure 4 illustrates a cross-sectional view of an exemplary medicated module attached to an exemplary drug delivery device;
Figure 5 illustrates a cross-sectional view of the exemplary medicated module and exemplary drug delivery device of Figure 4 after the capsule containing a second medicament is pierced; and
Figure 6 illustrates a cross-sectional view of the exemplary medicated module and exemplary drug delivery device of Figure 4 after the deformable membrane is pierced.

### DETAILED DESCRIPTION

Applicants' proposed concept is a system and method for dispensing a non-user settable dose of one medicament followed by a user-settable dose of a primary medicament using a single dispense interface. The proposed concept relates specifically to a method and system that utilizes a deformable membrane that facilitates first delivering all or substantially all of a given medicament through an output needle and then thereafter delivering yet another medicament through the same output needle in a single injection step.

A medicated module in accordance with embodiments of Applicants' proposed concepts may be attached to a primary drug delivery device, such as drug delivery device 100. Generally, Applicants' proposed medicated module includes a deformable membrane that facilitates sequential dosing of a secondary medicament followed by a primary medicament.

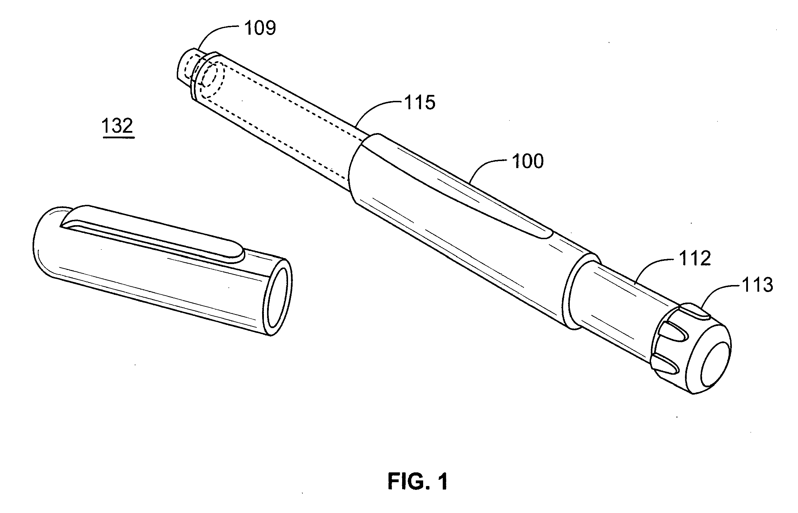

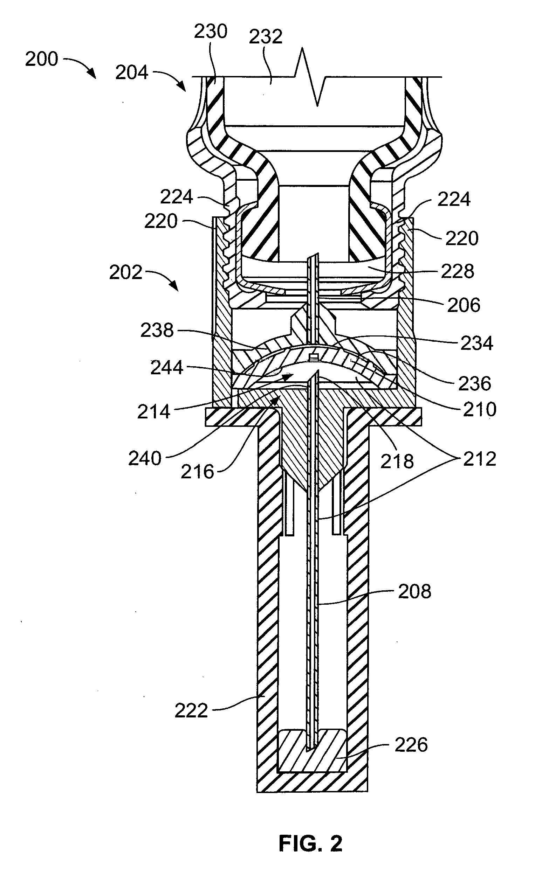

Figure 1 illustrates one example of a drug delivery device 100 that a medicated module, such as the medicated modules depicted in Figures 2-6, can be attached to. Specifically, the medicated module can be attached to the connection means 109 of distal end 132. A medicated module in accordance with Applicants' proposed concept is preferably self-contained and provided as a sealed and sterile disposable module that has an attachment means compatible to the attachment means 109 at the distal end 132 of device 100. Although not shown, the medicated module could be supplied by a manufacturer contained in a protective and sterile container, where the user would peel or rip open a seal or the container itself to gain access to the sterile medicated module. Further, the drug delivery device 100 includes a housing including a single dose setter 112. The dose setter 112 may be operably connected to a primary reservoir of medicament that may be stored in the drug delivery device, such as in cartridge holder 115. The user may use a dose dial button 113 in order to dial a user selectable dose of the primary medicament.

Applicants' proposed concept is a medicated module that is attachable to a drug delivery device such as drug delivery device 100 that has a drug reservoir holding a first, primary medicament. The medicated module includes a first needle, a second needle, and a deformable membrane located between the first and second needle. The medicated module also includes a second medicament that is located on a distal side of the deformable membrane. For example, the second medicament may be located in a cavity formed between the deformable membrane and a lower or bottom surface of the module. After the medicated module is attached to the drug delivery device, (i) the first needle is in fluid communication with the drug reservoir and (ii) the deformable membrane prevents fluid communication between the first and second needle. During dosing, the first medicament flows through the first needle and deforms the deformable membrane, wherein deformation of the deformable membrane forces substantially all of the second medicament to flow through the second needle. After substantially all of the second medicament is forced to flow through the second needle, the second needle pierces the deformable membrane, thereby opening up fluid communication between the first needle and the second needle. The first medicament may then be dispensed from the second needle. Thus, the deformable membrane facilitates sequential dosing of the second medicament followed by the first medicament, where the sequential dosing limits or prevents mixing of the medicaments until they have been injected.

Figure 2 illustrates a drug delivery system 200 including medicated module 202 and primary drug delivery device 204 that contains a first, primary medicament 232. In this Figure, only a partial view of the distal end of the primary drug delivery device 204 is shown. Primary drug delivery device 204 may be the same as or similar to drug delivery device 100 of Figure 1. The medicated module 202 includes a first needle 206 and a second needle 208. The first needle 206 may be referred to herein as an "engagement needle", as the needle engages with or communicates with the reservoir of drug delivery device 204 when the module and device are attached. Further, the second needle 208 may be referred to herein as an "output needle", as the second needle may be used to subcutaneously inject medicament into a user of drug delivery system 200.

The medicated module 202 also includes deformable member or membrane 210. It might be beneficial for the membrane to be produced from a material that is broadly inert when placed into long term contact with either the first or second medicament and that offers good performance with respect to leachables and / or extractables. Potential materials that this membrane could be manufactured from include, but are not limited to; TPE (Thermoplastic Elastomers), Liquid Silicone Rubber (LSR) and natural rubbers. Alternative materials, including Low-density Polyethylene (LDPE) or Linear low-density Polyethylene (LLDPE) are also possible. Where improved barrier properties are desirable, laminate materials may be used e.g. multilayer materials consisting of the primary membrane material (potentially as above) plus additional thin layers of materials like PVC (Polyvinyl chloride) PCTFE (Polychlorotrifluoro ethylene) or Aluminuim. As shown in Figure 2, at least part of the deformable membrane is located between the first needle 206 and the second needle 206. Thus, prior to injection, the deformable membrane prevents fluid communication between the first needle 206 and the second needle 208.

The medicated module may also contain second medicament 212. In this example, the second medicament is located in cavity 214 and in the second needle 208. As depicted, cavity 214 is a cavity that is formed by the deformable membrane 210 and a lower or bottom surface 216 of the medicated module 202. The second medicament 212 is located on a distal side of the deformable membrane 210. In this example, the piercing tip 218 of the second needle 218 extends into the cavity.

The medicated module 202 also includes an attachment means 220 and a needle cover 222. Attachment means 220 is configured to attach to a corresponding attachment means of a drug delivery device, such as attachment means 224 of drug delivery device 204 (or, in another example, the attachment means 109 at the distal end 132 of device 100). This needle cover 222 may have a connection feature (e.g., a snap-fit feature) that allows the cover to be removably attached to the body of the medicated module 202. Further, the needle cover 222 may include a seal 226 that is located in the needle cover. In a preferred embodiment, the seal 226 is located at a bottom base at the distal end of the needle cover. In another embodiment, the medicated module may include a retractable needle guard rather than a needle cover.

Figure 2 depicts the medicated module 202 after the module has been attached to drug delivery device 204 and prior to injection. Attachment of medicated module 202 to drug delivery device 204 causes the engagement needle 206 to penetrate the septum 228 of the drug cartridge or reservoir 230 of the drug delivery device 204. Once the engagement needle 206 has passed through the septum of the cartridge 230, fluid connection is made with the first, primary medicament 232. In other words, the first needle 206 is in fluid communication with the drug reservoir 230. At this stage, the deformable membrane 210 separates the first needle 206 and second needle 208, thereby preventing fluid communication between the two needles. Thus, the first medicament 232 does not interact with (e.g., mix with) second medicament 212.

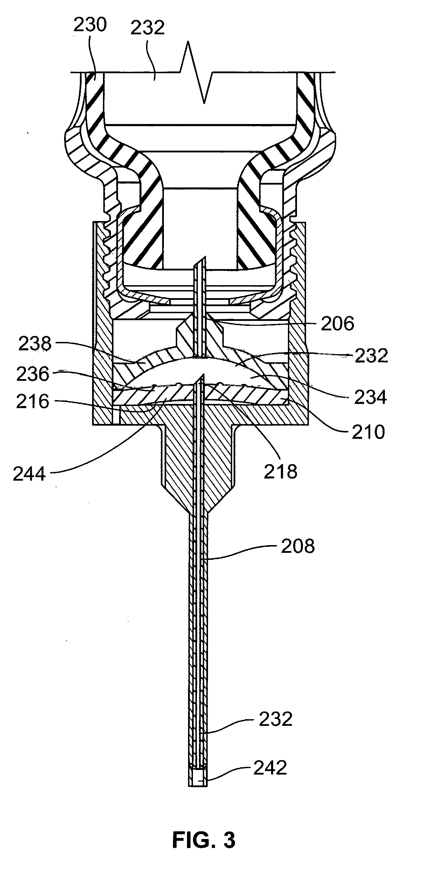

After the module 202 is attached to the device 204, a user may set a user-settable dose of the first medicament 232. The dose of the drug delivery device may be set in a usual manner (e.g., by dialing out an appropriate number of units of the primary medicament 232 with a dose dial). Dispense of the second medicament 212 followed by the first, primary medicament 232 may then be achieved via activation of the dosing mechanism of the drug delivery device. Dispense of the medicaments 212, 232 is described with reference to Figures 2 and 3.

As the primary medicament 232 is dispensed from the reservoir 230, the medicament 232 flows through the engagement needle 206 into a cavity 234 that is formed between the outer surface 236 (or proximal surface) of the deformable membrane 210 and medicament-retention feature 238. Retention feature 238 may also be referred to herein as a "retention cap", as the retention feature 238 forms a "cap" over the deformable membrane 210. The retention cap 238 is preferably substantially rigid compared with the deformable membrane 210. As an example, the retention cap may be composed of substantially rigid materials, such as an engineering polymer (e.g. Polypropylene - PP, Acrylonitrile Butadiene Styrene - ABS, Cyclo Olefin Polymer - COP, Polyethylene terephthalate - PET, Polycarbonate - PC, Polyoxymethylene - POM, Low Density Polyethylene - LDPE and others).

Since the retention cap 238 is substantially rigid and the first medicament 232 is generally or effectively incompressible, the deformable membrane 210 deforms as the first medicament 232 is displaced into the cavity 234. Specifically, the membrane 210 is deformed in such a way that a deformable portion of the membrane moves in the distal direction. The retention cap 238 is preferably fixed in the medicament module. Thus, the retention cap will not be moved when the first medicament 232 is forced into cavity 234. As shown, at least a portion of the retention cap 238 is located on the proximal side of the deformable membrane 210. Thus, the cap and membrane form the cavity 234 for receiving the primary medicament 232.

As seen when Figure 2 is compared to Figure 3, this deformation of the deformable membrane 210 in the distal direction forces second medicament 212 into and out the output needle 208. Specifically, deformation of the membrane causes the second medicament 212 to flow through an optional side hole 240 located in the output needle 208. The axis of the side hole may be perpendicular to the axis of the output needle and the side hole is preferably located close to the lower surface 216 of the fixed dose device 202. The medicament 212 is then forced through the needle 208 and out of output 242 at the distal end of the needle 208.

As described above, Applicants' side hole 240 is optional. As illustrated, initially the second medicament 212 will flow through the main inlet at the proximal end of the outlet needle 208 and the side hole 240. This would continue until the membrane 210 meets the tip of the needle 208 and is pierced. Without the side hole 240, this would mean that a small amount of residual second medicament 212 is left in the cavity. The presence of the optional side hole 240 helps ensure substantially all of the second medicament 212 gets dispensed under the action of the membrane 210.

At a predetermined deformation of the deformable membrane 210, the piercing tip 218 of the output needle 208, which protrudes above the lower surface 216 of the medicated module, comes to bear against the inside surface 244 (or distal surface) of the deformable membrane 210. Subsequent dispense of the first medicament 232 into the medicated module 202 then causes the deformable membrane 210 to be fully pierced or ruptured by the piercing tip 218 of the output needle 208. When the deformable membrane 210 has been pierced, fluid communication is established between the output needle 208 and the engagement needle 206. Since fluid communication is achieved between the output needle 208 and the engagement needle 206, the first medicament 232 may be dispensed through the output needle 208.

The predetermined deformation of the membrane that results in the piercing of the deformable membrane 210 preferably corresponds to when the second medicament 212 is substantially fully dispensed. In other words, when the output needle 208 pierces the deformable membrane 210 and opens up fluid communication between the two needles, substantially all of the second medicament preferably has been dispensed from the medicated module 202. It should be understood that although preferably substantially all of the second medicament 212 is dispensed before the primary medicament is being dispensed, it is not necessary that 100% of the second medicament is dispensed from the medicated module. For example, in an embodiment, approximately 85-90% of the medicament may be dispensed from the medicated module. Other examples are possible as well. Further, it should be understood that some of the second medicament 212 may still be in needle 208 when the needle 208 pierces the deformable member 210. Still further, a small amount of the second medicament 212 may still be in the cavity 214 when the needle 208 pierces the deformable member 210.

After the user finishes dispensing of the first medicament 232, the user may remove the output needle 208 from the injection site. Then, the depleted medicated module 202 may be disposed of. Assuming that the drug delivery device 204 still holds some first medicament 232, the drug delivery device 204 may be reused by the patient as required.

In an alternative embodiment, the deformable membrane may be a bi-stable membrane, such that the membrane is able to snap between the pre-dispensed state and the post-dispensed state. In this embodiment, when a small volume of the first, primary medicament is dispensed into the cavity formed between the deformable membrane and the retention cap, the membrane deforms by a sufficient amount to cause it to "snap" into its other stable state. Snapping into this other stable state may cause (i) the second medicament to be fully dispensed and (ii) the deformable membrane to be pierced by the piercing tip of the output needle.

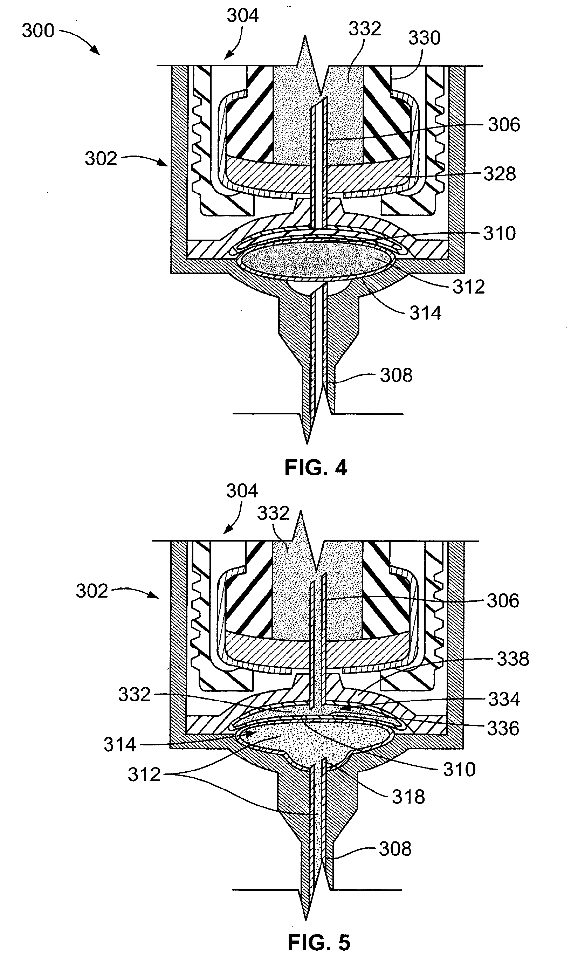

Another example medicated module is described with reference to Figures 4-6. This medicated module is similar in many respects to the medicated module 202 shown in Figures 2-3, and thus is not described in as great detail. However, this medicated module shown in Figures 4-6 includes a capsule 314 that contains the second medicament

In this example, the capsule 314 is located in a cavity. As depicted, the cavity is formed by the deformable membrane 310 and a lower or bottom surface of the medicated module 302. The second medicament 312 is located on a distal side of the deformable membrane 310. In this example, the piercing tip 318 of the second needle 318 extends into the cavity.

An exemplary benefit of containing the secondary medicament in a capsule is that it may be more straightforward to fill a capsule and assemble it into the device than to fill the cavity and needle of Figures 2-3 directly. Therefore, the manufacturing process of this embodiment may be less complex than the first embodiment. A capsule also has an additional benefit of offering the opportunity for improved sealing and vapour barrier properties compared with the example of Figures 2-3, as the capsule offers the opportunity for additional barrier layers and only becomes pierced during the dispense process.

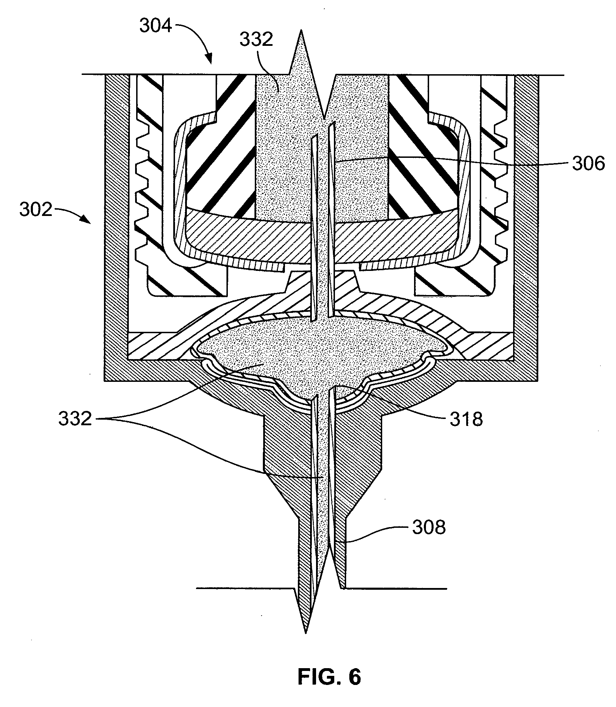

Figures 4-6 depict a drug delivery system 300 including medicated module 302 and drug delivery device 304. Specifically, Figure 4 depicts the module attached to the device prior to dispense, Figure 5 depicts the module and device as the secondary medicament begins to be dispensed, and Figure 6 depicts the module and device as the first, primary medicament is dispensed.

Medicated module 302 includes a first needle 306 (i.e., the engagement needle) and a second needle 308 (i.e., the output needle). The medicated module 302 also includes a deformable membrane 310. As shown in Figures 4-6, the deformable membrane 310 is located between the first needle 306 and the second needle 308 and sits adjacent an upper surface of the capsule 314. The second medicament 312 is located on a distal side of the deformable membrane 310. Prior to injection, this deformable membrane 310 works in conjunction with the capsule membrane 310 to prevent fluid communication between the two needles.

The medicated module also contains second medicament 312 that is located in a capsule 314. As an example, the capsule 314 may be composed of substantially rigid materials, such as an engineering polymer (e.g. Polypropylene - PP, Acrylonitrile Butadiene Styrene - ABS, Cyclo Olefin Polymer - COP, Polyethylene terephthalate - PET, Polycarbonate - PC, Polyoxymethylene - POM, Low Density Polyethylene - LDPE and others). As seen in Figure 4, prior to injection, the capsule 314 completely encloses and seals the second medicament 312. This capsule beneficially prevents or limits contamination of the second medicament, as the contents of the capsule are not exposed until the injection process begins. The capsule in the example of Figure 4 is depicted as an oval shape. However, it should be understood that the capsule may take a variety of other shapes as well (e.g., rectangular or circular).

When the medicated module 302 is attached to drug delivery device 304, the engagement needle 306 penetrates the septum 328 of the drug cartridge or reservoir 330 of the drug delivery device 304. Therefore, the engagement needle 306 is in fluid communication with the first, primary medicament 332 that is stored in drug reservoir 330.

After the module 302 is attached to the device 304, a user may set a dose. Similar to the example discussed with reference to Figures 2-3, the dose of the drug delivery device may be set in a usual manner (e.g., by dialing out an appropriate number of units of the primary medicament 332 with a dose dial). Dispense of the second medicament 312 followed by the first, primary medicament 332 may then be achieved.

With reference to Figure 5, as the primary medicament 332 is dispensed from reservoir 330, the medicament flows through the engagement needle 306 into a cavity 334 that is formed between the outer surface 336 (i.e., proximal surface) of the deformable membrane 310 and medicament-retention feature 338 (i.e., retention cap 338). Since the retention cap 338 is substantially rigid and the first medicament 332 is effectively incompressible, the deformable membrane 310 deforms as the first medicament 332 is displaced into the cavity 334. Specifically, a portion of the deformable membrane 310 deforms in the distal direction toward capsule 314.

One benefit of this arrangement is that it provides multiple thickness of barrier material between the primary and secondary medicaments. In addition, it reduces and/or removes the need for a fluid seal between the retention cap, 338, and the main body of the needle

This deformation of the membrane 310 forces the capsule 314 to be displaced in the distal direction, and the piercing tip 318 of the second needle 308 then pierces the lower surface of the capsule 314.. This piercing opens up fluid communication between the capsule 314 and the second needle 308. Therefore, medicament 312 may flow from the capsule out the output needle 308 as the deformable membrane forces the upper surface of the capsule 314 in the distal direction. That is, the capsule 314 essentially collapses under the influence of the expansion of the first capsule as it fills up with the primary medicament, thereby driving the secondary medicament 312 out of the outlet needle).

With reference to Figure 6, when substantially all of the second medicament 312 is dispensed from the capsule 314, the upper surface of the capsule 314 and then the lower surface of the second capsule 310 come into contact with the piercing tip 318. The tip 318 then pierces both membranes in sequence, opening up fluid communication between the second needle 308 and the first needle 306. Thus, the first medicament 332 may then be dispensed through the output needle 308. Figure 6 depicts the medicated module after these membrane surfaces have been pierced, and therefore first medicament 332 is being dispensed through the output needle 308. Preferably, these deformable membranes are not pierced until the capsule 314 is substantially collapsed under the force of the deformable membrane 310 and substantially all of the second medicament 312 has been depleted from the capsule 314.

Applicants' proposed concept also includes a method for dispensing a non-user settable dose of one medicament followed by a user-settable dose of a primary medicament. The method includes the step of attaching a medicated module to a drug delivery device, such as the medicated modules and drug delivery devices illustrated in Figures 1-6. As described above, the drug delivery device has a primary drug reservoir holding the primary medicament. Further, the medicated module includes a first needle, a second needle, a deformable membrane or capsule, and a second medicament. At least a portion of the deformable membrane is located between the first and second needle.. Further, the second medicament is located on a distal side of the deformable membrane.. After the medicated module is attached to the drug delivery device, (i) the first needle is in fluid communication with the primary drug reservoir and (ii) the deformable membrane prevents fluid communication between the first and second needle..

The method further includes the step of setting a dose of the primary medicament contained in the primary drug reservoir using a single dose setter of the drug delivery device.. The method also includes activating a dose button on the drug delivery device to cause the set dose of the primary medicament from the primary drug reservoir to flow in the distal direction toward the deformable membrane.. The primary medicament deforms the deformable membrane, and this deformation of the deformable membrane forces substantially all of the second medicament to flow through the second needle.. After substantially all of the second medicament is forced to flow through the second needle, the second needle pierces the deformable membrane, thereby opening up fluid communication between the first needle and the second needle.. The method then includes forcing the primary medicament to flow through the second needle.. Thus, the method includes sequential dosing of a second medicament followed by a first medicament.

As described above, Applicants' proposed concept beneficially allows for sequential dosing of a secondary medicament stored in a medicated module followed by a primary medicament from a primary drug delivery device.. The proposed deformable membrane ensures that the primary medicament is only delivered after the secondary medicament is substantially dispensed.. Thus, beneficially, the proposed concept prevents or limits the mixing of the two medicaments within the device and during delivery.. Avoidance of mixing of the 2 drug formulations during delivery might have several advantages. For example, it is known that the pharmacokinetics of certain drugs is critically dependent on their concentration. By delivering 2 drugs sequentially with no mixing the optimal concentration of each drug for optimal pharmacokinetics can be maintained. In addition, certain drugs have to be formulated in particular solvent environments (e.g., a specific pH range) to remain in solution. By delivering 2 drugs sequentially with no mixing the optimal pH range and therefore solubility can be maintained during delivery.

The connection or attachment between the medicated module of the above descried embodiments may contain additional features (not shown), such as connectors, stops, splines, ribs, grooves, and the like design features, that ensure that specific medicated module are attachable only to matching drug delivery devices.. Such additional features would prevent the insertion of a non-appropriate medicated module to a non-matching injection device.

The shape of the medicated module may be a cylindrical body or any other geometric shape suitable for defining a fluid reservoir or for containing discrete self-contained reservoir of the medicament in the medicated module and for attaching one or more needle cannula.. The integrated output needle can be any needle cannula suitable for subcutaneous or intramuscular injection.. Preferably the medicated module is provided by a drug manufacturer as a stand-alone and separate device that is sealed to preserve sterility.. The sterile seal of the module is preferably designed to be opened automatically, e.g. by cutting, tearing or peeling, when the medicated module is advanced or attached to the drug delivery device by the user.

The medicated module of Applicants' concept should be designed to operate in conjunction with a multiple use injection device, preferably a pen-type multi-dose injection device, similar to what is illustrated in Figure 1. The injection device could be a reusable or disposable device.. By disposable device it is meant an injection device that is obtained from the manufacturer preloaded with medicament and cannot be reloaded with new medicament after the initial medicament is exhausted.. The device may be a fixed dose or a settable dose and preferably a multi-dose device, however, in some cases it may be beneficial to use a single dose, disposable device.

A typical drug delivery device contains a cartridge or other reservoir of medication. This cartridge is typically cylindrical in shape and is usually manufactured in glass. The cartridge is sealed at one end with a rubber bung and at the other end by a rubber septum. The drug delivery pen is designed to deliver multiple injections.. The delivery mechanism is typically powered by a manual action of the user, however, the injection mechanism may also be powered by other means such as a spring, compressed gas or electrical energy.

Exemplary embodiments of the present invention have been described.. Those skilled in the art will understand, however, that changes and modifications may be made to these embodiments without departing from the true scope of the present invention, which is defined by the claims.

## Claims

1. A medicated module (202, 302) attachable to a drug delivery device, the drug delivery device having a drug reservoir holding a first medicament, the medicated module (202, 302) comprising:
a second needle (208, 308); and
a second medicament (212, 312);
wherein during dosing, substantially all of the second medicament is forced to flow through the second needle (208, 208);
**characterized in that** the medicated module (202, 302) further comprises:
a first needle (206, 306);
a deformable membrane (210, 310), wherein at least part of the deformable membrane (210, 310) is located between the first and second needle (206, 306; 208, 308);
wherein the second medicament (212, 312) is located on a distal side of the deformable membrane (210, 310),
wherein, after the medicated module (202, 302) is attached to the drug delivery device, (i) the first needle (206, 306) is in fluid communication with the drug reservoir and (ii) the deformable membrane (210, 310) prevents fluid communication between the first and second needle (206, 306; 208, 308);
wherein, during dosing, the first medicament flows through the first needle (206, 306) and deforms the deformable membrane (210, 310), wherein deformation of the deformable membrane (210, 310) forces substantially all of the second medicament (212, 312) to flow through the second needle (208, 308), and
wherein, after substantially all of the second medicament (212, 312) is forced to flow through the second needle (208, 308), the second needle (208, 308) pierces the deformable membrane (210, 310), thereby opening up fluid communication between the first and the second needle (206, 306; 208, 308).

2. The medicated module (202, 302) of claim 1, wherein the second medicament (212, 312) is located in at least one of (i) the second needle (208, 308) and (ii) a cavity (214) formed between the deformable membrane (210, 310) and the second needle (208, 308).

3. The medicated module (202, 302) of claim 2, wherein the cavity (214) is formed between the deformable membrane (210, 310) and a bottom surface (216) of the medicated module (202, 302).

4. The medicated module (202, 302) of claim 1, wherein the second medicament (212, 312) is located in a capsule (314) disposed between the deformable membrane (210, 310) and the second needle (208, 308).

5. The medicated module (202, 302) of claim 4, wherein the capsule (314) is sealed prior to dosing from the medicated module (202, 302).

6. The medicated module (202, 302) of claim 5, wherein, during dosing, the deformation of the deformable membrane (210, 310) causes the second needle (208, 308) to pierce the capsule (314), thereby opening up fluid communication between the second needle (208, 308) and the capsule (314).

7. The medicated module (202, 302) of any of the preceding claims, further comprising:
a medicament-retention feature (238, 338), wherein at least a portion of the medicament-retention feature (238, 338) is located on a proximal side of the deformable membrane (210, 310), wherein the medicament-retention feature (238, 338) and an outer surface (236, 336) of the deformable membrane (210, 310) form a cavity (234, 334) that holds the first medicament that flows through the first needle (206, 306), wherein the first medicament in the cavity (234, 334) deforms the deformable membrane (210, 310).

8. The medicated module (202, 302) of claim 7, wherein the medicament-retention feature (238, 338) is fixed in the medicament module (202, 302).

9. The medicated module (202, 302) of claim 7, wherein the medicament-retention feature (238, 338) comprises a substantially rigid material so that the medicament-retention feature (238, 338) is not deformable.

10. The medicated module (202, 302) of any of the preceding claims, wherein the second needle (208, 308) pierces the deformable membrane (210, 310) after a pre-determined deformation of the deformable membrane (210, 310).

11. The medicated module (202, 302) of claim 10, wherein the pre-determined deformation corresponds to the second medicament (212, 312) being substantially fully dispensed from the medicated module (202, 302).

12. The medicated module (202, 302) of any of the preceding claims, wherein a piercing tip (218, 318) of the second needle (208, 308) pierces the deformable membrane (210,310).

13. The medicated module of any of the preceding claims, wherein the deformable membrane (210, 310) is a bi-stable deformable membrane, wherein the bi-stable membrane snaps from a pre-dispense state to a post-dispense state after a sufficient amount of deformation, wherein the second needle (208, 308) pierces the bi-stable deformable membrane as the bi-stable deformable membrane snaps into the post-dispense state.

## Patentansprüche

1. Arzneistoff enthaltendes Modul (202, 302), das an einer Arzneimittelabgabevorrichtung befestigt werden kann, wobei die Arzneimittelabgabevorrichtung einen ein erstes Medikament enthaltenden Arzneimittelbehälter aufweist, wobei das Arzneistoff enthaltende Modul (202, 302) Folgendes umfasst:
eine zweite Nadel (208, 308); und
ein zweites Medikament (212, 312);
worin während der Dosierung im Wesentlichen das gesamte zweite Medikament gezwungen wird, durch die zweite Nadel (208, 308) zu fließen;
**dadurch gekennzeichnet, dass** das Arzneistoff enthaltende Modul (202, 302) ferner Folgendes umfasst:
eine erste Nadel (206, 306);
eine verformbare Membran (210, 310), worin zumindest ein Teil der verformbaren Membran (210, 310) zwischen der ersten und der zweiten Nadel (206, 306; 208, 308) angeordnet ist;
worin das zweite Medikament (212, 312) auf einer distalen Seite der verformbaren Membran (210, 310) angeordnet ist,
worin nach der Befestigung des Arzneistoff enthaltenden Moduls (202, 302) an der Arzneimittelabgabevorrichtung (i) die erste Nadel (206, 306) mit dem Arzneimittelbehälter in Fluidverbindung steht, und (ii) die verformbare Membran (210, 310) eine Fluidverbindung zwischen der ersten und der zweiten Nadel (206, 306; 208, 308) verhindert;
worin während der Dosierung das erste Medikament durch die erste Nadel (206, 306) fließt und die verformbare Membran (210, 310) verformt, worin die Verformung der verformbaren Membran (210, 310) im Wesentlichen das gesamte zweite Medikament (212, 312) zwingt, durch die zweite Nadel (208, 308) zu fließen, und
worin, nachdem im Wesentlichen das gesamte zweite Medikament (212, 312) gezwungen wurde, durch die zweite Nadel (208, 308) zu fließen, die zweite Nadel (208, 308) die verformbare Membran (210, 310) durchsticht, wodurch die Fluidverbindung zwischen der ersten und der zweiten Nadel (206, 306; 208, 308) geöffnet wird.

2. Arzneistoff enthaltendes Modul (202, 302) nach Anspruch 1, worin das zweite Medikament (212, 312) zumindest in (i) der zweiten Nadel (208, 308) und (ii) einem zwischen der verformbaren Membran (210, 310) und der zweiten Nadel (208, 308) geformten Hohlraum (214) angeordnet ist.

3. Arzneistoff enthaltendes Modul (202, 302) nach Anspruch 2, worin der Hohlraum (214) zwischen der verformbaren Membran (210, 310) und einer Unterseite (216) des Arzneistoff enthaltenden Moduls (202, 302) geformt ist.

4. Arzneistoff enthaltendes Modul (202, 302) nach Anspruch 1, worin das zweite Medikament (212, 312) in einer Kapsel (314) angeordnet ist, die zwischen der verformbaren Membran (210, 310) und der zweiten Nadel (208, 308) angeordnet ist.

5. Arzneistoff enthaltendes Modul (202, 302) nach Anspruch 4, worin die Kapsel (314) vor der Dosierung aus dem Arzneistoff enthaltendem Modul (202, 302) verschlossen wird.

6. Arzneistoff enthaltendes Modul (202, 302) nach Anspruch 5, worin während der Dosierung die Verformung der verformbaren Membran (210, 310) die zweite Nadel (208, 308) veranlasst, die Kapsel (314) zu durchstechen, wodurch die Fluidverbindung zwischen der zweiten Nadel (208, 308) und der Kapsel (314) geöffnet wird.

7. Arzneistoff enthaltendes Modul (202, 302) nach einem der vorhergehenden Ansprüche, ferner umfassend:
ein Medikament-Rückhaltemittel (238, 338), worin zumindest ein Teil des Medikament-Rückhaltemittels (238, 338) auf einer proximalen Seite der verformbaren Membran (210, 310) angeordnet ist, worin das Medikament-Rückhaltemittel (238, 338) und eine Außenseite (236, 336) der verformbaren Membran (210, 310) einen Hohlraum (234, 334) bilden, der der das erste Medikament hält, das durch die erste Nadel (206, 306) fließt, worin das erste Medikament in dem Hohlraum (234, 334) die verformbaren Membran (210, 310) verformt.

8. Arzneistoff enthaltendes Modul (202, 302) nach Anspruch 7, worin das Medikament-Rückhaltemittel (238, 338) an dem Arzneistoff enthaltenden Modul (202, 302) fixiert ist.

9. Arzneistoff enthaltendes Modul (202, 302) nach Anspruch 7, worin das Medikament-Rückhaltemittel (238, 338) ein im Wesentlichen starres Material umfasst, so dass das Medikament-Rückhaltemittel (238, 338) nicht verformbar ist.

10. Arzneistoff enthaltendes Modul (202, 302) nach einem der vorhergehenden Ansprüche, worin die zweite Nadel (208, 308) nach einer vorbestimmten Verformung der verformbaren Membran (210, 310) die verformbare Membran (210, 310) durchsticht.

11. Arzneistoff enthaltendes Modul (202, 302) nach Anspruch 10, worin die vorbestimmte Verformung der im Wesentlichen vollständigen Abgabe des zweiten Medikaments (212, 312) aus dem Arzneistoff enthaltendem Modul (202, 302) entspricht.

12. Arzneistoff enthaltendes Modul (202, 302) nach einem der vorhergehenden Ansprüche, worin eine Einstechspitze (218, 318) der zweiten Nadel (208, 308) die verformbare Membran (210, 310) durchsticht.

13. Arzneistoff enthaltendes Modul nach einem der vorhergehenden Ansprüche, worin die verformbare Membran (210, 310) eine bistabile verformbare Membran ist, worin die bistabile Membran nach einem ausreichenden Verformungsgrad von einem Zustand vor Abgabe in einen Zustand nach Abgabe schnappt, worin die zweite Nadel (208, 308) die bistabile durchstechbare Membran durchsticht, wenn die bistabile verformbare Membran in den Zustand nach Abgabe schnappt.

## Revendications

1. Module (202, 302) médicamenteux pouvant être fixé sur un dispositif d'administration de médicament, le dispositif d'administration de médicament étant doté d'un réservoir de médicament contenant un premier médicament, le module (202, 302) médicamenteux comportant :
une seconde aiguille (208, 308) ; et
un second médicament (212, 312) ;
dans lequel durant le dosage, sensiblement la totalité du second médicament est forcée de s'écouler dans la seconde aiguille (208, 308) ;
**caractérisé en ce que** le module (202, 302) médicamenteux comporte en outre :
une première aiguille (206, 306) ;
une membrane (210, 310) déformable, dans lequel au moins une partie de la membrane (210, 310) déformable est située entre les première et seconde aiguilles (206, 306 ; 208, 308) ;
dans lequel le second médicament (212, 312) est situé sur un côté distal de la membrane (210, 310) déformable,
dans lequel, une fois que le module (202, 302) médicamenteux est fixé au dispositif d'administration de médicament, (i) la première aiguille (206, 306) est en communication fluidique avec le réservoir de médicament (ii) et la membrane (210, 310) déformable empêche une communication fluidique entre les première et seconde aiguilles (206, 306 ; 208, 308) ;
dans lequel, durant le dosage, le premier médicament s'écoule dans la première aiguille (206, 306) et déforme la membrane (210, 310) déformable, la déformation de la membrane (210, 310) déformable forçant sensiblement la totalité du second médicament (212, 312) à s'écouler dans la seconde aiguille (208, 308) et
dans lequel, une fois que sensiblement la totalité du second médicament (212, 312) est forcée de s'écouler dans la seconde aiguille (208, 308), la seconde aiguille (208, 308) perce la membrane (210, 310) déformable, ouvrant ainsi une communication fluidique entre les première et seconde aiguilles (206, 306 ; 208, 308).

2. Module (202, 302) médicamenteux selon la revendication 1, dans lequel le second médicament (212, 312) est situé dans au moins (i) la seconde aiguille (208, 308) et (ii) une cavité (214) formée entre la membrane (210, 310) déformable et la seconde aiguille (208, 308).

3. Module (202, 302) médicamenteux selon la revendication 2, dans lequel la cavité (214) est formée entre la membrane (210, 310) déformable et une surface (216) inférieure du module (202, 302) médicamenteux.

4. Module (202, 302) médicamenteux selon la revendication 1, dans lequel le second médicament (212, 312) est situé dans une capsule (314) disposée entre la membrane (210, 310) déformable et la seconde aiguille (208, 308).

5. Module (202, 302) médicamenteux selon la revendication 4, dans lequel la capsule (314) est fermée hermétiquement avant le dosage dans le module (202, 302) médicamenteux.

6. Module (202, 302) médicamenteux selon la revendication 5, dans lequel, durant le dosage, la déformation de la membrane (210, 310) déformable amène la seconde aiguille (208, 308) à percer la capsule (314), ouvrant ainsi une communication fluidique entre la seconde aiguille (208, 308) et la capsule (314).

7. Module (202, 302) médicamenteux selon l'une quelconque des revendications précédentes, comportant en outre :
une caractéristique (238, 338) de retenue du médicament, dans lequel au moins une partie de la caractéristique (238, 338) de retenue du médicament est située sur un côté proximal de la membrane (210, 310) déformable, dans lequel la caractéristique (238, 338) de retenue du médicament et une surface (236, 336) externe de la membrane (210, 310) déformable forment une cavité (234, 334) qui contient le premier médicament s'écoulant dans la première aiguille (206, 306), le premier médicament dans la cavité (234, 334) déformant la membrane (210, 310) déformable.

8. Module (202, 302) médicamenteux selon la revendication 7, dans lequel la caractéristique (238, 338) de retenue du médicament est fixée dans le module (202, 302) médicamenteux.

9. Module (202, 302) médicamenteux selon la revendication 7, dans lequel la caractéristique (238, 338) de retenue du médicament comporte un matériau sensiblement rigide de sorte que la caractéristique (238, 338) de retenue du médicament n'est pas déformable.

10. Module (202, 302) médicamenteux selon l'une quelconque des revendications précédentes, dans lequel la seconde aiguille (208, 308) perce la membrane (210, 310) déformable après une déformation prédéterminée de la membrane (210, 310) déformable.

11. Module (202, 302) médicamenteux selon la revendication 10, dans lequel la déformation prédéterminée correspond au second médicament (212, 312) qui est sensiblement entièrement distribué depuis le module (202, 302) médicamenteux.

12. Module (202, 302) médicamenteux selon l'une quelconque des revendications précédentes, dans lequel une pointe (218, 318) de perçage de la seconde aiguille (208, 308) perce la membrane (210, 310) déformable.

13. Module médicamenteux selon l'une quelconque des revendications précédentes, dans lequel la membrane (210, 310) déformable est une membrane déformable bistable, dans lequel la membrane bistable passe en s'encliquetant d'un état de prédistribution à un état de postdistribution après une quantité suffisante de déformation, la seconde aiguille (208, 308) perçant la membrane déformable bistable au fur et à mesure que la membrane déformable bistable s'encliquette dans la position de postdistribution.
